# EUROPEAN PATENT SPECIFICATION

(11) **EP 3 735 194 B1**
(45) Date of publication and mention of the grant of the patent: **10.07.2024**
(21) Application number: 18842837.9
(22) Date of filing: 18.12.2018
(51) Int. Cl.: A61B 18/18

(54) **SYSTEMS AND METHODS FOR ENERGY DELIVERY**
SYSTEME UND VERFAHREN ZUR ENERGIEABGABE
SYSTÈMES ET PROCÉDÉS D'APPLICATION D'ÉNERGIE

(30) Priority: 03.01.2018 US 201862613301 P; 14.12.2018 US 201816221154
(43) Date of publication of application: 11.11.2020
(73) Proprietor: Neuwave Medical, Inc., Madison, WI 53704 (US)
(72) Inventor: THOM, Mark, Madison, Wisconsin 53704 (US)
(74) Representative: Murgitroyd & Company
(86) International application number: PCT/IB2018/060238
(87) International publication number: WO 2019/135135

(56) References cited:
- EP-A1- 3 461 448
- WO-A1-2010/048334
- WO-A2-2011/017168
- US-A1- 2007 249 999

## Description

### FIELD OF INVENTION

The present invention relates to a microwave ablation probe and to a system including such a probe. The probe and system may allow delivery of energy to tissue for a wide variety of applications, including medical procedures (e.g., tissue ablation, resection, cautery, vascular thrombosis, treatment of cardiac arrhythmias and dysrhythmias, electrosurgery, tissue harvest, etc.).

### BACKGROUND

Energy delivery devices (e.g., antennas, probes, electrodes, etc.) (e.g., microwave ablation devices) (e.g., radiofrequency ablation devices) are used to deliver energy to a desired tissue region for purposes of "treating" a desired tissue region. Ablation therapy (e.g., microwave ablation, radiofrequency ablation) is a widely used, minimally invasive technique for the treatment of various conditions and/or disorders (e.g., tumor cells). Within such techniques, ablation energy (e.g., microwave energy) (e.g., radiofrequency (RF) energy) is used to heat a desired tissue region to a desired temperature to cause tissue destruction in the heated region.

In some situations, laparoscopic surgery is used for ablation. Laparoscopic surgery requires several small incisions in or near a desired tissue region for the insertion of trocars or small cylindrical tubes approximately 5 to 10 millimeters in diameter through which surgical instruments (e.g., microwave ablation devices) and a laparoscope are placed into the tissue region and/or cavity. The laparoscope illuminates the surgical field and sends a magnified image from inside the body to a video monitor giving a surgeon a close-up view of the organs and tissues. The surgeon watches the live video feed and performs the operation by manipulating the surgical instruments placed through the trocars.

Traditional laparoscopic surgery can utilize the same probe used in percutaneous procedures. Percutaneous probes are generally constructed using a 15 to 25cm long rigid shaft that can limit maneuverability in laparoscopic procedures.

Similarly for robotic laparoscopic surgeries, it is possible to use a robot to place percutaneous probes, however, the rigid shaft of the probe limits access to desired target areas. Robotic surgery typically utilizes RF energy instead of microwave energy. Several different clamp or jaw tools capable of forming factors with integrated RF surgical tools are available. Such integrated RF tools are widely used. However, the cumbersome nature and limited mobility of rigid percutaneous microwave ablation probes has limited the adoption of microwave technology for robotic and traditional laparoscopic procedures.

WO 2011/017168 A2 describes comprehensive systems, devices and methods for delivering energy to tissue for a wide variety of applications, including medical procedures (e.g., tissue ablation, resection, cautery, vascular thrombosis, treatment of cardiac arrhythmias and dysrhythmias, electrosurgery, tissue harvest, etc.). Systems, devices, and methods are provided for treating a tissue region (e.g., a tumor) through application of energy.

WO 2010/048334 A1 describes medical systems and methods adapted for the delivery of various medical components such as microwave antennas within or on a body for performing one or more medical procedures. Medical systems are described comprising a combination of one or more medical components and one or more elongate steerable or non-steerable arms that are adapted to mechanically manipulate the one or more medical components. Several microwave antennas are described that comprise an additional diagnostic or therapeutic modality located on or in the vicinity of the microwave antennas.

Improved probes for use in laparoscopic ablation are needed.

The present invention addresses this need.

### SUMMARY

According to an aspect of the invention, there is provided a microwave ablation probe according to claim 1.

According to an aspect of the invention, there is provided a system according to claim 8.

Further features according to embodiments are defined in the dependent claims.

Provided herein are microwave ablation probes and systems that solve the problem of maneuvering rigid laparoscopic probes during procedures involving microwave ablation. The microwave ablation probes described herein comprise a flexible component for easy maneuverability and a rigid component (e.g., comprising a plurality of metal protrusions) for easy grasping by robotic or manual grasping tools. Such microwave ablation probes provide for more robust and accurate ablation procedures.

Such microwave ablation probes are not limited to particular positioning of the microwave emission section, the rigid section, and the flexible section. In some embodiments, the microwave emission section is positioned distal to the rigid section, and the rigid section is positioned distal to the flexible section. In some embodiments, the rigid section comprises one or more protrusions configured to engage with a grasping portion of a laparoscopic instrument. In some embodiments, the microwave emission section is configured to emit microwave energy.

Such microwave ablation probes are not limited to a specific microwave emission section. In some embodiments, the microwave emission section includes a microwave antenna. In some embodiments, the microwave antenna comprises a co-axial transmission line. In some embodiments, the microwave antenna comprises a tri-axial transmission line.

Such microwave ablation probes are not limited to a specific rigid section. In some embodiments, the rigid section is between approximately 2 to 10 cm in length. In some embodiments, the rigid section has one or more (e.g., one, two, three, four, five or more) protrusions configured to engage with a grasping portion of a laparoscopic instrument. In some embodiments, the one or more protrusions configured to engage with a grasping portion of a laparoscopic instrument are metal protrusions.

In some embodiments, the grasping portion of such a laparoscopic instrument is a slot or recess configured to detachably accept the protrusions. For example, in some embodiments, the protrusion is configured such that it can detachably fit into such a slot or recess for purposes of detachably engaging the microwave ablation probe with the laparoscopic instrument.

In some embodiments, the shape of the protrusion is configured to match a variety of known laparoscopic instruments having known slots or recesses configured to detachably accept protrusions having a specific shape (e.g., geometry).

In some embodiments, the shape of the protrusion is configured to match a specific laparoscopic instruments having known slots or recesses configured to detachably accept protrusions having a specific shape (e.g., geometry).

It is understood that other detachable mechanisms may be used for detachable engagement between the grasping portion and the protrusions for purposes of detachably engaging the microwave ablation probe with the laparoscopic instrument (e.g., cantilever-type snaps or the like). In some embodiments, the grasping portion of such a laparoscopic instrument is a slotted jaw.

In some embodiments, the laparoscopic instrument is manually operable. In some embodiments, the laparoscopic instrument is robotically operable. In some embodiments, the systems further comprise a power supply electrically connected to the microwave ablation probe.

Methods of ablating a tissue region are disclosed but not claimed, comprising providing a laparoscopic instrument having a grasping portion and the described microwave ablation probe, grasping the one or more protrusions of the microwave ablation probe with the grasping portion of the laparoscopic instrument, positioning the microwave emission section of the microwave ablation probe at a desired tissue location, and delivering microwave energy to the desired tissue location with the microwave ablation probe under conditions such that the desired tissue region is ablated. The tissue region may be within a subject (e.g., a human subject).

### BRIEF DESCRIPTON OF THE DRAWINGS

FIG. 1 shows an exemplary microwave ablation probe for laparoscopic use.
FIGS. 2, 3 and 4 show a microwave ablation probe engaged with a laparoscopic instrument.

### DETAILED DESCRIPTION

The present invention relates to a microwave ablation probe and to a system including such a probe. The probe and system may allow delivery of energy to tissue for a wide variety of applications, including medical procedures (e.g., tissue ablation, resection, cautery, vascular thrombosis, treatment of cardiac arrhythmias and dysrhythmias, electrosurgery, tissue harvest, etc.). The microwave ablation probe comprises flexible and rigid sections.

Currently used ablation probes are rigid, and therefore difficult to manipulate or maneuver during a procedure involving ablation of a tissue region. A need exists for flexible microwave ablation probes having rigid distal end portions configured for engagement with laparoscopic instruments (e.g., manual or robotic). The present disclosure addresses this need by providing microwave ablation probes with both rigid (e.g., configured for facilitating engagement with laparoscopic instruments (e.g., manual or robotic)) and flexible sections.

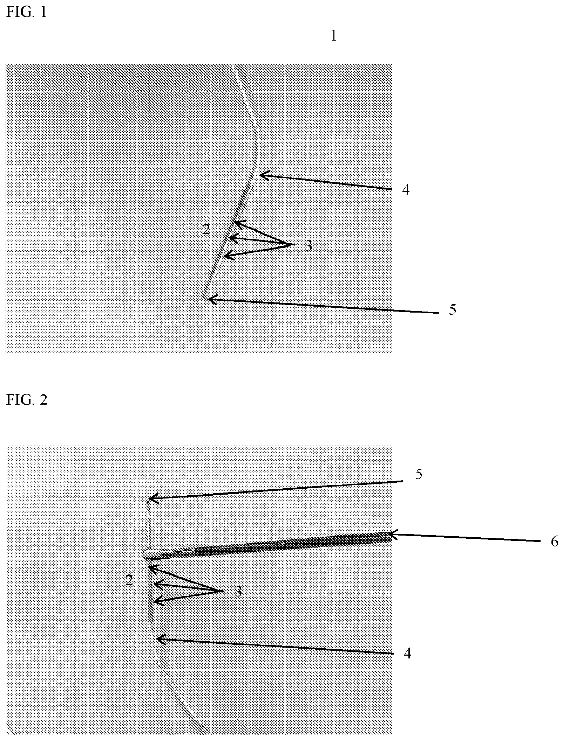

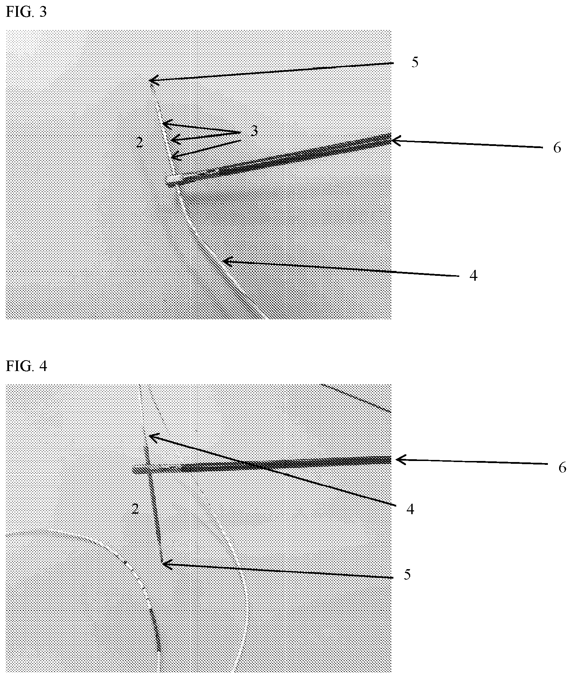

Accordingly, provided herein are microwave ablation probes (e.g., for use in laparoscopic ablation procedures) comprising both rigid and flexible sections. Exemplary probes are described in Figures 1-4.

Figure 1 shows a microwave ablation probe **1** configured for facilitating engagement with laparoscopic instruments (e.g., manual or robotic) while maintaining flexibility. As shown in Fig. 1, the microwave ablation probe **1** has a rigid section **2** (having protrusions **3),** a flexible section **4,** and a microwave emission section **5.**

Still referring to Fig. 1, the rigid section **2** is not limited to specific dimensions and characteristics. For example, in some embodiments, the rigid section **2** is configured such that it is able to facilitate engagement (e.g., securing, attaching) with a laparoscopic instrument (e.g., manual or robotic). The rigid section **2** is not limited to a particular width and length. In some embodiments, the width and length of the rigid section **2** is such that it does not impede function of the microwave ablation probe **1** during an ablation procedure. In some embodiments, the width of the rigid section **2** is approximately between 0.1 and 2 cm (e.g., 0.1, 0.2, 0.5, 0.8, 0.9, 1, 1.5, 1.85, 1.99, 2, 2.01, 2.1, 3 cm). In some embodiments, the length of the rigid section **2** is approximately between 2 to 10 cm (e.g., 0.5, 1, 1.3, 1.5, 1.8, 1.999, 2, 3, 4, 5, 6, 7, 8, 9, or 10, 10.1, 10.3, 10.5, 10.8, 11, 15, etc, cm or a fraction thereof).

Still referring to Fig.1, the rigid section **2** further contains protrusions **3** configured for engagement (e.g., securing, attaching) with a laparoscopic instrument (e.g., manual or robotic). The rigid section **2** is not limited to having a particular number of protrusions **3.** In some embodiments, the rigid section **2** can from 1 to 50 protrusions **3.** In some embodiments, the rigid section **2** can have from 1 to 50 protrusions **3.** In some embodiments as shown in Fig. 1, the rigid section **2** has 7 protrusions **3.**

Still referring to Fig. 1, the protrusions **3** are not limited to specific dimensions and characteristics. For example, in some embodiments, the protrusions **3** are configured such that engagement (e.g., securing, attaching) of the microwave ablation probe **1** with a laparoscopic instrument (e.g., manual or robotic) is facilitated. The protrusions **3** are not limited to a particular width and length. In some embodiments, the width and length of the protrusions **3** are such that it does not impede function of the microwave ablation probe **1** during an ablation procedure. In some embodiments, the width and length of each of the protrusions **3** are identical. In some embodiments, the width and length of each of the protrusions **3** are non-identical. In some embodiments, the length of a protrusion **3** is approximately between 0.1 and 10 cm (e.g., 0.1, 0.2, 0.5, 0.8, 0.9, 1, 1.5, 1.85, 1.99, 2, 2.01, 2.1, 3, 4, 5, 6, 7, 8, 9, 10, 11, 12 cm). In some embodiments, the width of a protrusion **3** is between approximately 0.1 and 2 cm (e.g., 0.1, 0.2, 0.5, 0.8, 0.9, 1, 1.5, 1.85, 1.99, 2, 2.01, 2.1, 3 cm). The protrusions **3** are not limited to a specific composition. In some embodiments, the protrusions **3** are metal. The protrusions **3** are not limited to a particular positioning along the rigid section **2.** In some embodiments, as shown in Fig.1, the protrusions **3** are positioned along the entire rigid section **2.** In some embodiments, the protrusions **3** are positioned along only a portion (e.g., 99%, 90%, 80%, 60%, 50%, 25%, 10%, 1%) of the rigid section **2.**

Still referring to Fig. 1, the protrusions **3** are not limited to a specific composition. For example, in some embodiments, the protrusions **3** are metal. In some embodiments, the protrusions **3** are plastic. In some embodiments, the protrusions **3** are ceramic. In some embodiments, the protrusions **3** are a mixture of different kinds of metal and/or plastic.

Still referring to Fig. 1, the protrusions **3** are configured for detachable engagement with the grasping portion of such a laparoscopic instrument. For example, in some embodiments, the protrusion **3** is configured such that it can detachably fit into a grasping portion having the shape of a slot or recess for purposes of detachably engaging the microwave ablation probe with the laparoscopic instrument.

It is understood that other detachable mechanisms may be used for detachable engagement between the grasping portion and the protrusions **3** for purposes of detachably engaging the microwave ablation probe with the laparoscopic instrument (e.g., cantilever-type snaps or the like). In some embodiments, the grasping portion of such a laparoscopic instrument is a slotted jaw.

Still referring to Fig. 1, the flexible section **4** is not limited to specific dimensions and characteristics. For example, in some embodiments, the flexible section **4** is configured such that it is able to be maneuvered in a flexible manner while the rigid section **2** is engaged with a laparoscopic instrument (e.g., manual or robotic). The flexible section **4** is not limited to a particular width and length. In some embodiments, the width and length of the flexible section **4** is such that it does not impede function of the microwave ablation probe **1** during an ablation procedure. In some embodiments, the width of the flexible section **4** is approximately between 0.1 and 2 cm (e.g., 0.1, 0.2, 0.5, 0.8, 0.9, 1, 1.5, 1.85, 1.99, 2, 2.01, 2.1, 3 cm). In some embodiments, the length of the flexible section **4** is any desired length (e.g., approximately 1 cm to 10,000 cm). The flexible section **4** is not limited to a particular positioning along the microwave ablation probe **1.** In some embodiments, the flexible section **4** is located at the proximal end of the microwave ablation probe **1.** In some embodiments, the flexible section **4** is located along the entire length of the microwave ablation probe **1** not occupied by the rigid section **2** and the microwave emission section **5.**

Still referring to Fig. 1, the microwave emission section **5** is not limited to specific dimensions and characteristics. For example, in some embodiments, the microwave emission section **5** is configured such that it is able to emit microwave energy while the rigid section **2** is engaged with a laparoscopic instrument (e.g., manual or robotic). The microwave emission section **5** is not limited to a particular width and length. In some embodiments, the width and length of the microwave emission section **5** is such that it does not impede function of the microwave ablation probe **1** during an ablation procedure. In some embodiments, the width of the microwave emission section **5** is approximately between 0.1 and 2 cm (e.g., 0.1, 0.2, 0.5, 0.8, 0.9, 1, 1.5, 1.85, 1.99, 2, 2.01, 2.1, 3 cm). In some embodiments, the length of the microwave emission section **5** is approximately between 2 to 10 cm (e.g., 0.5, 1, 1.3, 1.5, 1.8, 1.999, 2, 3, 4, 5, 6, 7, 8, 9, or 10, 10.1, 10.3, 10.5, 10.8, 11, 15, etc, cm or a fraction thereof).

The microwave emission section **5** is not limited to a particular positioning along the microwave ablation probe **1.** As shown in Fig. 1, the microwave emission section **5** is located at the most distal end of the microwave ablation probe **1.** In some embodiments, the microwave emission section **5** is a microwave antenna. As such, in some embodiments, positioning of the rigid section **2** at the distal end of a microwave ablation probe **1** permits localization of the rigid section **2** in the immediate vicinity of the microwave emission section **5.** In some embodiments wherein both the rigid section **2** and the microwave emission section **5** are distally positioned along the microwave ablation probe **1,** the microwave emission section **5** is more distally positioned than the rigid section **2** (as shown in Fig. 1). In some embodiments wherein both the rigid section **2** and the microwave emission section **5** are distally positioned along the microwave ablation probe **1,** the rigid section **2** directly abuts the microwave emission section **5.** In some embodiments wherein both the rigid section **2** and the microwave emission section **5** are distally positioned along the microwave ablation probe **1,** a gap (e.g., 0.1 cm, 0.25 cm, 0.5 cm, 0.8 cm, 1 cm, 1.5 cm, 5 cm, 10 cm, etc) exists between the rigid section **2** and the microwave emission section **5.**

The microwave ablation probes are not limited to a particular manner of emitting microwave energy. As shown in Fig. 1, the microwave ablation probes have a microwave emission section. In some embodiments, the microwave emission section has therein antennae configured to emit microwave energy. Such microwave ablation probes are not limited to particular types or designs of antennae (e.g., ablation device, surgical device, etc.). In some embodiments, the systems utilize energy delivery devices having linearly shaped antennae (see, e.g., U.S. Patent Nos. 6,878,147, 4,494,539, U.S. Patent Application Serial Nos. 11/728,460, 11/728,457, 11/728,428, 10/961,994, 10/961,761; and International Patent Application No., WO 03/039385). In some embodiments, the systems utilize energy delivery devices having non-linearly shaped antennae (see, e.g., U.S. Patent Nos. 6,251,128, 6,016,811, and 5,800,494, U.S. Patent Application Serial No. 09/847,181, and International Patent Application No. WO 03/088858). In some embodiments, the antennae have horn reflection components (see, e.g., U.S. Patent Nos. 6,527,768, 6,287,302). In some embodiments, the antenna has a directional reflection shield (see, e.g., U.S. Patent No. 6,312,427).

In some embodiments, the antennae within microwave ablation probes as defined herein have a coaxial transmission line configuration. Such microwave ablation probes are not limited to particular configurations of coaxial transmission lines. Examples of coaxial transmission lines include, but are not limited to, coaxial transmission lines developed by Pasternack, Micro-coax, and SRC Cables. In some embodiments, the coaxial transmission line has a center conductor, a dielectric element, and an outer conductor (e.g., outer shield). In some embodiments, such microwave ablation probes utilize antennae having flexible coaxial transmission lines (e.g., for purposes of positioning around, for example, pulmonary veins or through tubular structures) (see, e.g., U.S. Patent Nos. 7,033,352, 6,893,436, 6,817,999, 6,251,128, 5,810,803, 5,800,494). In some embodiments, such microwave ablation probes utilize antennae having rigid coaxial transmission lines (see, e.g., U.S. Patent No. 6,878,147, U.S. Patent Application Serial Nos. 10/961,994, 10/961,761, and International Patent Application No. WO 03/039385).

In some embodiments, the antennae within microwave ablation probes as defined herein have a triaxial transmission line configuration. Such microwave ablation probes are not limited to particular configurations of triaxial transmission lines. In some embodiments, the triaxial transmission line is configured with optimized tuning capabilities (see, e.g., U.S. Patent No. 7,101,369; see, also, U.S. Patent Application Nos. 10/834,802, 11/236,985, 11/237,136, 11,237,430, 11/440,331, 11/452,637, 11/502,783, 11/514,628; and International Patent Application No. PCT/US05/14534).

Figs. 2, 3 and 4 show a microwave ablation probe **1** engaged with a laparoscopic instrument (e.g., manual or robotic) **6.** As shown, the laparoscopic instrument (e.g., manual or robotic) **6** is engaged with one of the protrusions **3** positioned along the rigid section **2** of the microwave ablation probe **1.** As shown, the laparoscopic instrument **6** is shown engaging the one of the protrusions **3** positioned along the rigid section **2** of the microwave ablation probe **1** via a grasping portion (e.g., slotted jaws). As can be seen, such an engagement is not hindering the flexibility of the flexible section **4.** As can be seen, the microwave emission section **5** is positioned at the most distal end of the microwave ablation probe **1.**

The present invention is not limited to use with a particular type of laparoscopic instrument. In some embodiments, the laparoscopic instrument is manually operable. In some embodiments, the laparoscopic instrument is robotically operable. Examples of laparoscopic instruments that can engage with a microwave ablation probe of the present invention include, but are not limited to, cannulas and trocars, trocar incision closure devices, electrodes and electrosurgical cables, laparoscopic bipolar scissors and graspers, forceps and graspers, hooks and probes, knot pushers, needles and needle holders, rigid scopes, retractors, and scissors. In some embodiments, the laparoscopic instrument is the Da Vinci robot (Intuitive Surgical).

The microwave ablation probes of the present invention may be combined within various system/kit embodiments. For example, in some embodiments, systems and/or kits comprising one or more or microwave ablation probes and one or more laparoscopic instruments (e.g., manual or robotic) are provided. In some embodiments, the present invention provides systems and/or kits comprising one or more or microwave ablation probes and one or more of the following: laparoscopic instruments (e.g., manual or robotic), a computer having a processor, a generator, a power distribution system, and an energy applicator, along with any one or more accessory component (e.g., surgical instruments, temperature monitoring devices, imaging systems, etc.) are provided. Exemplary system components are described in U.S. Pat. Nos. 7,101,369, 9,072,532, 9,119,649, and 9,192,438 and U.S. Publ. No. 20130116679.

The systems of the present invention may be used in any medical procedure involving delivery of microwave energy to a tissue region.

The systems are not limited to treating a particular type or kind of tissue region (e.g., brain, liver, heart, blood vessels, foot, lung, bone, etc.). The systems may, for instance, find use in ablating tumor regions. Additional treatments include, but are not limited to, treatment of heart arrhythmia, tumor ablation (benign and malignant), control of bleeding during surgery, after trauma, for any other control of bleeding, removal of soft tissue, tissue resection and harvest, treatment of varicose veins, intraluminal tissue ablation (e.g., to treat esophageal pathologies such as Barrett's Esophagus and esophageal adenocarcinoma), treatment of bony tumors, normal bone, and benign bony conditions, intraocular uses, uses in cosmetic surgery, treatment of pathologies of the central nervous system including brain tumors and electrical disturbances, sterilization procedures (e.g., ablation of the fallopian tubes) and cauterization of blood vessels or tissue for any purposes. The surgical application may comprise ablation therapy (e.g., to achieve coagulative necrosis). The surgical application may comprise tumor ablation to target, for example, primary or metastatic tumors. The surgical application may comprise the control of hemorrhage (e.g. electrocautery). The surgical application may comprise tissue cutting or removal.

In some embodiments, the energy delivery systems utilize processors that monitor and/or control and/or provide feedback concerning one or more of the components of the system. In some embodiments, the processor is provided within a computer module. For example, in some embodiments, the systems provide software for regulating the amount of microwave energy provided to a tissue region through monitoring one or more characteristics of the tissue region including, but not limited to, the size and shape of a target tissue, the temperature of the tissue region, and the like (e.g., through a feedback system) (see, e.g., U.S. Patent Application Serial Nos. 11/728,460, 11/728,457, and 11/728,428). In some embodiments, the software is configured to provide information (e.g., monitoring information) in real time. In some embodiments, the software is configured to interact with the energy delivery systems such that it is able to raise or lower (e.g., tune) the amount of energy delivered to a tissue region. In some embodiments, the software is designed to regulate coolant. The type of tissue being treated (e.g., liver) may be inputted into the software for purposes of allowing the processor to regulate (e.g., tune) the delivery of energy to the tissue region based upon pre-calibrated methods for that particular type of tissue region. In some embodiments, the processor may be operable to generate a chart or diagram based upon a particular type of tissue region displaying characteristics useful to a user of the system. In some embodiments, the processor may be operable to provide energy delivering algorithms for purposes of, for example, slowly ramping power to avoid tissue cracking due to rapid out-gassing created by high temperatures. In some embodiments, the processor may be operable to allow a user to choose power, duration of treatment, different treatment algorithms for different tissue types, simultaneous application of power to the antennas in multiple antenna mode, switched power delivery between antennas, coherent and incoherent phasing, etc. In some embodiments, the processor is configured for the creation of a database of information (e.g., required energy levels, duration of treatment for a tissue region based on particular patient characteristics) pertaining to ablation treatments for a particular tissue region based upon previous treatments with similar or dissimilar patient characteristics. In some embodiments, the processor is configured to be operated by remote control.

In some embodiments, user interface software is provided for monitoring and/or operating the components of the energy delivery systems. In some embodiments, the user interface software is operable by a touch screen interface. In some embodiments, the user interface software may be implemented and operated within a sterile setting (e.g., a procedure room) or in a non-sterile setting. In some embodiments, the user interface software is implemented and operated within a procedure device hub (e.g., via a processor). In some embodiments, the user interface software is implemented and operated within a procedure cart (e.g., via a processor). The user interface software is not limited to particular functions. Examples of functions associated with the user interface software include, but are not limited to, tracking the number of uses per component within the energy delivery system (e.g., tracking the number of times an energy delivery device is used), providing and tracking real time temperatures of each component or parts of each component (e.g., providing real time temperature of different locations along an energy delivery device (e.g., at the handle, at the stick, at the tip)) (e.g., providing real time temperature of the cables associated with the energy delivery systems), providing and tracking real time temperature of the tissue being treated, providing an automatic shut off for the part or all of the energy delivery system (e.g., an emergency shut off), generation of reports based upon the data accumulated, for example, prior to, during and after a procedure, providing audible and/or visual alerts to a user (e.g., alerts indicating a procedure has begun and/or is finished, alerts indicating a temperature has reached an aberrant level, alerts indicating the length of the procedure has gone beyond a default, etc.).

In some embodiments, the energy delivery systems utilize imaging systems comprising imaging devices. The energy delivery systems are not limited to particular types of imaging devices (e.g., endoscopic devices, stereotactic computer assisted neurosurgical navigation devices, thermal sensor positioning systems, motion rate sensors, steering wire systems, intraprocedural ultrasound, interstitial ultrasound, microwave imaging, acoustic tomography, dual energy imaging, fluoroscopy, computerized tomography magnetic resonance imaging, nuclear medicine imaging devices triangulation imaging, thermoacoustic imaging, infrared and/or laser imaging, electromagnetic imaging) (see, e.g., U.S. Patent Nos. 6,817,976, 6,577,903, and 5,697,949, 5,603,697, and International Patent Application No. WO 06/005,579). In some embodiments, the systems utilize endoscopic cameras, imaging components, and/or navigation systems that permit or assist in placement, positioning, and/or monitoring of any of the items used with the energy systems of the present invention.

In some embodiments, the energy delivery systems utilize tuning elements for adjusting the amount of energy delivered to the tissue region. In some embodiments, the tuning element is manually adjusted by a user of the system. In some embodiments, a tuning system is incorporated into an energy delivery device so as to permit a user to adjust the energy delivery of the device as desired (see, e.g., U.S. Patent Nos. 5,957969, 5,405,346).

In some embodiments, the energy delivery systems utilize coolant systems so as to reduce undesired heating within and along an energy delivery device (e.g., tissue ablation catheter). The systems are not limited to a particular cooling system mechanism.

In some embodiments, the energy delivering systems utilize temperature monitoring systems. In some embodiments, temperature monitoring systems may be used to monitor the temperature of an energy delivery device (e.g., with a temperature sensor). In some embodiments, temperature monitoring systems may be used to monitor the temperature of a tissue region (e.g., tissue being treated, surrounding tissue). In some embodiments, the temperature monitoring systems are designed to communicate with a processor for purposes of providing temperature information to a user or to the processor to allow the processor to adjust the system appropriately.

The system may further employ one or more additional components that either directly or indirectly take advantage of or assist the features of the present invention. For example, in some embodiments, one or more monitoring devices may be used to monitor and/or report the function of any one or more components of the system. Additionally, any medical device or system that might be used, directly or indirectly, in conjunction with the devices of the present invention may be included with the system. Such components include, but are not limited to, sterilization systems, devices, and components, other surgical, diagnostic, or monitoring devices or systems, computer equipment, handbooks, instructions, labels, and guidelines, robotic equipment, and the like.

The systems are not limited to particular uses. Indeed, the energy delivery systems of the present invention are designed for use in any setting wherein the emission of energy is applicable. Such uses include any and all medical, veterinary, and research applications. In addition, the systems and devices of the present invention may be used in agricultural settings, manufacturing settings, mechanical settings, or any other application where energy is to be delivered. In some embodiments, the systems are configured for open surgery, percutaneous, intravascular, intracardiac, endoscopic, intraluminal, laparoscopic, or surgical delivery of energy. In some embodiments, the energy delivery devices may be positionable within a patient's body through a catheter, through a surgically developed opening, and/or through a body orifice (e.g., mouth, ear, nose, eyes, vagina, penis, anus) (e.g., a N.O.T.E.S. procedure). In some embodiments, the systems are configured for delivery of energy to a target tissue or region. In some particular embodiments, the probes described herein may find use in laparoscopic procedures.

The present invention is not limited by the nature of the target tissue or region. Uses include, but are not limited to, treatment of heart arrhythmia, tumor ablation (benign and malignant), control of bleeding during surgery, after trauma, for any other control of bleeding, removal of soft tissue, tissue resection and harvest, treatment of varicose veins, intraluminal tissue ablation (e.g., to treat esophageal pathologies such as Barrett's Esophagus and esophageal adenocarcinoma), treatment of bony tumors, normal bone, and benign bony conditions, intraocular uses, uses in cosmetic surgery, treatment of pathologies of the central nervous system including brain tumors and electrical disturbances, sterilization procedures (e.g., ablation of the fallopian tubes) and cauterization of blood vessels or tissue for any purposes. In some embodiments, the surgical application comprises ablation therapy (e.g., to achieve coagulative necrosis). In some embodiments, the surgical application comprises tumor ablation to target, for example, metastatic tumors. In some embodiments, the device is configured for movement and positioning, with minimal damage to the tissue or organism, at any desired location, including but not limited to, the brain, neck, chest, abdomen, and pelvis. In some embodiments, the systems are configured for guided delivery, for example, by computerized tomography, ultrasound, magnetic resonance imaging, fluoroscopy, and the like.

Methods of treating a tissue region are disclosed but nor claimed, comprising providing a tissue region and a system described herein (e.g., a microwave ablation probe, and optionally a power supply, a temperature monitor, an imager, a tuning system, and/or a temperature reduction system); and performing ablation used the system. The tissue region may be a tumor. The delivering of the energy may result in, for example, the ablation of the tissue region and/or thrombosis of a blood vessel, and/or electroporation of a tissue region. The tissue region may be a tumor. The tissue region may comprise one or more of the heart, liver, genitalia, stomach, lung, large intestine, small intestine, brain, neck, bone, kidney, muscle, tendon, blood vessel, prostate, bladder, and spinal cord.

## Claims

1. A microwave ablation probe (1), comprising:
a microwave emission section (5), a rigid section (2), and a flexible section (4),
wherein the microwave emission section is positioned distal to the rigid section,
wherein the rigid section is positioned distal to the flexible section,
wherein the rigid section comprises one or more protrusions (3) configured to engage with a grasping portion of a laparoscopic instrument (6),
wherein the microwave emission section is configured to emit microwave energy.

2. The microwave ablation probe of Claim 1, wherein the microwave emission section is a microwave antenna.

3. The microwave ablation probe of Claim 2, wherein the microwave antenna comprises a co-axial transmission line.

4. The microwave ablation probe of Claim 2, wherein the microwave antenna comprises a tri-axial transmission line.

5. The microwave ablation probe of Claim 1, wherein the rigid section comprises is between approximately 2 to 10 cm in length.

6. The microwave ablation probe of Claim 1, wherein the rigid section has five or more protrusions configured to engage with the grasping portion of a laparoscopic instrument.

7. The microwave ablation probe of Claim 1, wherein the one or more protrusions configured to engage with the grasping portion of a laparoscopic instrument are metal protrusions.

8. A system comprising:
the microwave ablation probe of any preceding claim, and
said laparoscopic instrument.

9. The system of Claim 8, wherein the grasping portion of the laparoscopic instrument is a slotted jaw.

10. The system of Claim 8, wherein the laparoscopic instrument is manually operable.

11. The system of Claim 8, wherein the laparoscopic instrument is robotically operable.

12. The system of Claim 10, further comprising a power supply electrically connected to the microwave ablation probe.

## Patentansprüche

1. Mikrowellenablationssonde (1), umfassend:
einen Mikrowellenemissionsabschnitt (5), einen starren Abschnitt (2) und einen flexiblen Abschnitt (4),
wobei der Mikrowellenemissionsabschnitt distal zu dem starren Abschnitt positioniert ist,
wobei der starre Abschnitt distal zu dem flexiblen Abschnitt positioniert ist,
wobei der starre Abschnitt einen oder mehrere Vorsprünge (3) umfasst, die konfiguriert sind, mit einem Greifteil eines laparoskopischen Instruments (6) in Eingriff zu kommen,
wobei der Mikrowellenemissionsabschnitt konfiguriert ist, Mikrowellenenergie zu emittieren.

2. Mikrowellenablationssonde nach Anspruch 1, wobei der Mikrowellenemissionsabschnitt eine Mikrowellenantenne ist.

3. Mikrowellenablationssonde nach Anspruch 2, wobei die Mikrowellenantenne eine Koaxialübertragungsleitung bzw. Koaxialleitung umfasst.

4. Mikrowellenablationssonde nach Anspruch 2, wobei die Mikrowellenantenne eine Triaxialübertragungsleitung bzw. Triaxialleitung umfasst.

5. Mikrowellenablationssonde nach Anspruch 1, wobei der starre Abschnitt eine Länge zwischen etwa 2 und 10 cm aufweist.

6. Mikrowellenablationssonde nach Anspruch 1, wobei der starre Abschnitt fünf oder mehr Vorsprünge aufweist, die konfiguriert sind, mit dem Greifteil eines laparoskopischen Instruments in Eingriff zu kommen.

7. Mikrowellenablationssonde nach Anspruch 1, wobei der eine oder die mehreren Vorsprünge, die konfiguriert sind, mit dem Greifteil eines laparoskopischen Instruments in Eingriff zu kommen, Metallvorsprünge sind.

8. System, umfassend:
die Mikrowellenablationssonde nach einem der vorhergehenden Ansprüche, und
das laparoskopische Instrument.

9. System nach Anspruch 8, wobei der Greifteil des laparoskopischen Instruments eine geschlitzte Backe bzw. Backe mit Schlitzen ist.

10. System nach Anspruch 8, wobei das laparoskopische Instrument manuell bedienbar ist.

11. System nach Anspruch 8, wobei das laparoskopische Instrument robotergestützt bedienbar ist.

12. System nach Anspruch 10, ferner umfassend eine Leistungsversorgung, die elektrisch mit der Mikrowellenablationssonde verbunden ist.

## Revendications

1. Sonde d'ablation par hyperfréquences (1), comprenant :
une section d'émission d'hyperfréquences (5), une section rigide (2) et une section flexible (4),
dans laquelle la section d'émission d'hyperfréquences est positionnée de manière distale à la section rigide,
dans laquelle la section rigide est positionnée de manière distale à la section flexible,
dans laquelle la section rigide comprend une ou plusieurs saillies (3) configurées pour venir en prise avec une partie de saisie d'un instrument laparoscopique (6),
dans laquelle la section d'émission d'hyperfréquences est configurée pour émettre une énergie hyperfréquences.

2. Sonde d'ablation par hyperfréquences selon la revendication 1, dans laquelle la section d'émission d'hyperfréquences est une antenne hyperfréquences.

3. Sonde d'ablation par hyperfréquences selon la revendication 2, dans laquelle l'antenne hyperfréquences comprend une ligne de transmission coaxiale.

4. Sonde d'ablation par hyperfréquences selon la revendication 2, dans laquelle l'antenne hyperfréquences comprend une ligne de transmission triaxiale.

5. Sonde d'ablation par hyperfréquences selon la revendication 1, dans laquelle la section rigide mesure approximativement 2 à 10 cm de long.

6. Sonde d'ablation par hyperfréquences selon la revendication 1, dans laquelle la section régie présente cinq saillies ou plus configurées pour venir en prise avec la partie de saisie d'un instrument laparoscopique.

7. Sonde d'ablation par hyperfréquences selon la revendication 1, dans laquelle les une ou plusieurs saillies configurées pour venir en prise avec la partie de saisie d'un instrument laparoscopique sont des saillies métalliques.

8. Système comprenant :
la sonde d'ablation par hyperfréquences selon une quelconque revendication précédente, et
ledit instrument laparoscopique.

9. Système selon la revendication 8, dans lequel la partie de saisie de l'instrument laparoscopique est un mors rainuré.

10. Système selon la revendication 8, dans lequel l'instrument laparoscopique est actionnable manuellement.

11. Système selon la revendication 8, dans lequel l'instrument laparoscopique est actionnable de manière robotisée.

12. Système selon la revendication 10, comprenant en outre une alimentation électrique reliée électriquement à la sonde d'ablation par hyperfréquences.
